# EUROPÄISCHE PATENTANMELDUNG

(11) **EP 0 576 895 A2**
(43) Veröffentlichungstag der Anmeldung: **05.01.1994**
(21) Anmeldenummer: 93109495.7
(22) Anmeldetag: 14.06.1993
(51) Int. Cl.: C12N 1/20, C02F 3/34, C02F 3/12

(54) **Verfahren zum mikrobiellen Abbau substituierter Aromaten**

(30) Priorität: 16.06.1992 DE 4219638
(71) Anmelder: Pascik, Imre, Dr., D-40789 Monheim (DE)
(72) Erfinder: Glancer, Margarete, Prof. Dr., HR-41000 Zagreb (HR); Ban, Sinisa, Prof. Dr., HR-41000 Zagreb (HR); Pascik, Imre, Dr., D-40789 Monheim (DE)

(57) **Zusammenfassung**

Verfahren zum mikrobiellen Abbau substituierter Aromaten dadurch gekennzeichnet, daß dieser mit Hilfe einer Mikroorganismenmischkultur bestehend aus den Stämmen
Pseudomonas desmolytica
Bacillus cereus
Bacillus circulans und
Flavobacterium devorans
erfolgt, wobei gleichzeitig mit dem Abbau von organischen Verbindungen gleichzeitig eine Nitrifikation von Amino-, bzw. Ammoniumstickstoff zum Nitrat stattfindet

## Beschreibung

Die Erfindung betrifft ein Verfahren zum oxidativen mikrobiellen Abbau von mono-, di- oder höher-substituierten Aromaten, insbesondere von substituierten Arylmono-, Aryldi- und Aryltrisulfonsäuren bzw. deren Kondensationsprodukte. Von besonderer Bedeutung sind dabei wiederum Verbindungen, die als Substituenten Amino-und/oder Ammonstickstoff-abspaltende Gruppen enthalten.

Die mikrobielle Umwandlung von substituierten Aromaten in Industrieabwässern gewinnt in den letzten Jahren zunehmend an Bedeutung. Eine wichtige Rolle spielt dabei der Abbau substituierter Arylsulfonsäuren, wie z. B. substituierter Benzolsulfonsäuren, Benzoldisulfonsäuren, Naphthalinsulfonsäuren, Naphthalindisulfonsäuren, Naphthalintrisulfonsäuren und Ligninsulfonsäuren.

In dem Buch "Microbial Degradation of Xenobiotics and Recalcitrant Compounds" von T. Leisinger, A. M. Cook, R. Hütter und J. Nüesch, Academic Press, London, 1981, wird auf Seite 149 erwähnt, daß der Abbau verschiedener Aromaten über Katechole, in erster Linie über 1,2-Dihydroxybenzol, bzw. Cis-benzene-glycol, abläuft, die dann weiter aerob bzw. anaerob metabolisiert werden.

In der Veröffentlichung von C. Brilon und H. J. Knackmuss: "Enrichment and Isolation of Naphthalenesulfonic Acid - utilizing Pseudomonas" in "Applied and Environmental Microbiology" Juli 1981, Seite 39 bis 43, wird über den Bioabbau von substituierten Naphthalinsulfonsäuren mit zwei isolierten Pseudomonas-Stämmen berichtet, wobei mit speziellen Nährböden gearbeitet wurde. Außerdem wurden Spurenelemente sowie die Abbauendprodukte Pyruvat, Succinat bzw. Vitamin B 12 und Hefeextrakt zugesetzt, wobei nicht näher definierte Intermediäre entstehen, die durch Bakterien verwertet werden.

Es wurde gefunden, daß der mikrobielle Abbau von substituierten Arylmono-, Aryldi- und Aryltrisulfonsäuren, insbesondere von Naphthalinmono-, Naphthalindi- und Naphthalintrisulfonsäuren mit Hilfe einer Mischkultur, bestehend aus den Stämmen Pseudomonas, Bacillus und Flavobacterium, bei einem pH-Wert von 6,5 bis 8,5, Temperaturen von 15 °C bis 35 °C und unter Zugabe unterstöchiometrischer Mengen an Sauerstoff auch ohne Zusatz besonderer Spurenelemente bzw. Vitamine gelingt.

Gute Ergebnisse werden erzielt, wenn die genannten Gattungen zu Beginn der Reaktion (Startbedingungen) in folgender prozentualer Verteilung vorliegen:
- Pseudomonas: 35 % bis 60 %, bevorzugt 40 % bis 55 %, besonders bevorzugt 45 % bis 50 %
- Flavobacterium: 5 % bis 20 %, bevorzugt 6 % bis 15 %, besonders bevorzugt 7 % bis 12 %
- Bacillus: 25 % bis 50 %, bevorzugt 30 % bis 45 %, besonders bevorzugt 35 % bis 40 %
Als besonders aktiv hat sich eine Mischkultur erwiesen, bestehend aus den Stämmen:
1. Pseudomonas desmolytica, mit der Bezeichnung LOV 853 (4/5) der Mikroorganismensammlung des Instituts für Biotechnologie der Universität in Zagreb (Croatien), beziehungsweise gemäß Hinterlegung unter der Nummer DSM 5779 in der Deutschen Sammlung von Mikroorganismen und Zellkulturen (DSM) in D-3300 Braunschweig, und
1.1 Pseudomonas desmolytica, mit der Bezeichnung LOV 848 (4/4), beziehungsweise gemäß Hinterlegung unter der Nummer DSM 6738, bei der DSM, zu je 50 %, mit einem prozentuellen Anteil zum Beginn des Bioabbaus von 35 % bis 60 %, bevorzugt 40 % bis 55 %, besonders bevorzugt 45 % bis 50 %,
2. Bacillus cereus, mit der Bezeichnung LOV 832 (4/1), beziehungsweise gemäß Hinterlegung unter der Nummer DSM 5766, bei der DSM sowie
2.1 Bacillus circulans mit der Bezeichnung LOV 904 (4/9), beziehungsweise gemäß Hinterlegung unter der Nummer DSM 6983, bei der DSM, mit einem gemeinsamen prozentuellen Anteil von 25 % bis 50 %, bevorzugt 30 % bis 45 %, besonders bevorzugt 35 % bis 40 %, und
3. Flavobacterium devorans, mit der Bezeichnung LOV 866 (4/6), beziehungsweise gemäß Hinterlegung unter der Nummer DSM 5773, bei der DSM, mit einem prozentuellen Anteil von 5 % bis 20 %, bevorzugt 6 % bis 15 % sowie besonders bevorzugt 7 % bis 12 %.

Vorzugsweise wird bei Temperaturen von 20 °C bis 30 °C, besonders bevorzugt 23 °C bis 28 °C und bei einem pH-Wert von 6,8 bis 7,6, besonders bevorzugt von 7,0 bis 7,4 gearbeitet.

Die Zudosierung unterstöchiometrischer Mengen von Sauerstoff erfolgt vorteilhaft in der Weise, daß eine Konzentration an gelöstem Sauerstoff von 0,05 bis 3 mg/l, bevorzugt 0,1 bis 2,0 mg/l und besonders bevorzugt 0,2 bis 0,9 mg/l vorhanden ist.

Weiterhin kann in manchen Fällen eine Verbesserung der Abbauleistung erzielt werden, wenn dem Substrat bzw. dem zu reinigenden Abwasser bis zu 10 mg/l KH₂PO₄, MgSO₄ sowie CaCl₂ und FeCl₃ zugegeben werden.

Überraschenderweise wurde ferner gefunden, daß die erfindungsgemäßen Mischkulturen unter den angegebenen Bedingungen im Medium bzw. im Abwasser vorhandene Amino- und/oder Ammonstickstoffgruppen bis zum Nitrat nitrifizieren. Dies bedeutet, daß der Abbau der Arylsulfonsäuren gleichzeitig mit einer Nitrifikation einhergeht. Eine simultane Spaltung bzw. Öffnung der aromatischen Ringe und Nitrifikation bis zum Nitrat waren nach dem Stand der Technik nicht zu erwarten, zumal bei der Nitrifikation normalerweise mit einem wesentlich höheren Sauerstoffeintrag gearbeitet wird, außerdem ist die Gegenwart der nitrifizierten Bakterien Nitrosomonas und Nitrobacter obligatorisch.

Wenn keine stickstoffhaltigen Gruppen vorhanden sind, können die zuletzt beschriebenen vorteilhaften Prozeßbedingungen durch Zugabe von Ammoniumverbindungen oder Ammonium-abspaltenden Verbindungen, wie z. B. Harnstoff, gewährleistet werden.

Nach dem bisherigen Stand der Technik wurde bei vergleichbaren Problemlösungen hauptsächlich mit Reinkulturen bzw. aus Mischungen von 1 bis 3 Reinkulturen unter Zusatz von Feinchemikalien, Vitaminen, Hefeextrakt und Spurenelementen gearbeitet. Dabei ergaben sich häufig lange Adaptationszeiten bis zu mehreren Monaten. Häufig wurde auch beobachtet, daß die Reaktion nicht in der gewünschten Richtung abläuft. Diese Nachteile werden mit dem erfindungsgemäßen Verfahren überwunden.

Durch die Anwendung einer aus der Umgebung der Abbauprodukte selektierten, aus mehreren Stämmen zusammengesetzten Mischkultur, wird diese robuster, benötigt keine Feinchemikalien und Vitamine. Auch auf den Zusatz von Spurenelementen kann in vielen Fällen verzichtet werden. Ein besonderer Vorteil der erfindungsgemäßen Mischkultur liegt in ihrer größeren Selektivität, was insbesondere bei Molekülen, die mehrere verschiedene Substituenten beinhalten, wie z. B. die 6-Amino-2-naphthalinsulfonsäure, zum Tragen kommt. Durch die Zusammensetzung der Mischkultur und Variation der Arbeitsbedingungen in den angegebenen Bereichen kann man nicht nur die Elimination eines oder zweier Substituenten, sondern auch die zeitliche Reihenfolge der Elimination einzelner Substituenten steuern. Dieser Effekt wurde vor allem bei der Verwendung von 6-Amino-2-naphthalinsulfonsäure als Substrat beobachtet.

Nach dem Stand der Technik wird bei der Umwandlung dieser Substanz in Gegenwart von gelöstem O₂ zunächst die -SO₃H-Gruppe aus dem Molekül abgespalten, was dazu führt, daß es in weiteren Reaktionsschritten zur Oligomerisierung des Restmoleküls über die Aminogruppe zu rot bis braun gefärbten Oligomeren kommt, wodurch die weitere Umwandlung bzw. Metabolisierung gehemmt wird.

Dagegen wird bei dem erfindungsgemäßen Verfahren zunächst die -NH₂-Gruppe gezielt abgespalten, so daß es im weiteren Verlauf des Metabolismus nicht zur Akkumulation von hemmenden Intermediären kommt.

Überraschenderweise erweist sich die erfindungsgemäße Mischkultur unter Anwendung des erfindungsgemäßen Verfahrens als fähig, sowohl die Oxidation von Kohlenstoffverbindungen bis zum Kohlendioxid, als auch von NH₄-N- enthaltenden oder NH₄-N-abspaltenden Verbindungen bis zum Nitrat zu oxidieren, und zwar unter derart hoher CSB- und Ammoniumschlammbelastung, ausgedrückt als Gramm Substrat per Gramm Biomasse und Tag, unter denen eine Nitrifikation mit einem "üblichen" nitrifizierenden Belebtschlamm nicht möglich ist.

Die erfindungsgemäße Mischkultur kann sowohl in diskontinuierlich als auch halbkontinuierlich und kontinuierlich betriebenen Reaktoren eingesetzt werden, wobei die Zusammensetzung der Mischkultur durch die Selektion sehr lange gleichmäßig bleibt.

Bei dem Betrieb von biologischen Kläranlagen kommt es in der Praxis oft zu Schwankungen, die sich besonders im Fall von Schwankungen der Substratzusammensetzungen störend auf die Leistung der Anlage auswirken, wobei die für den Abbau bestimmter Abwasserinhaltsstoffe nötigen Stämme geschädigt und aus dem Reaktor gespült werden können.

Um solchen Störungen vorzubeugen, wird in einer bevorzugten Verfahrensvariante die selektierte Mischkultur in einem separaten Fermenter angezüchtet und in kleinen Mengen kontinuierlich oder halbkontinuierlich in den Bioreaktor der Kläranlage zudosiert.

Bei dem erfindungsgemäßen Verfahren und besonders bei der erfindungsgemäßen Mischkultur handelt es sich um eine Erfindung, die in ihrer Neuheit und Wirksamkeit den bisherigen Stand der Technik übertrifft. Während in klassischen Kläranlagen die Biomasse manchmal mehrere Jahre braucht, um sich auf die persistenten Stoffe zu adaptieren, braucht die gezielte Selektion und Züchtung des Inokulums nur wenige Wochen bis zum Erreichen einer weitgehenden Eliminationsleistung.

Die folgenden Beispiele dienen zur Erläuterung und Verdeutlichung der Vorteile des erfindungsgemäßen Verfahrens.

### Beispiel 1

Eine Lösung von 500 mg/l 6-Amino-2-naphthalinsulfonsäure in 1 000 ml Leitungswasser wird nach Zusatz von 10 ml der wässrigen Suspension einer Mikroorganismenmischkultur, bestehend ursprünglich aus
30 % Pseudomonas desmolytica (1 : 1-Mischung aus den Stämmen DSM 5779 und DSM 6738),
30 % Flavobacterium devorans (DSM 5773) und
40 % Micrococcus
und Zusatz von 10 mg/l KH₂PO₄ sowie Spuren von CaCl₂ und FeCl₃ bei pH = 7 und 25 °C in einem Erlenmeyer-Kolben unter Verwendung eines Magnetrührers derart belüftet, daß das Reaktionsmedium einen Gehalt an gelöstem Sauerstoff von DO = 2 bis 4 mg/l O₂ auf-weist. Nach wenigen Stunden beginnt sich der Kolbeninhalt leicht gelb zu verfärben. Eine HPLC-Analyse des Mediums nach 12 h Reaktionszeit ergab einen Gehalt von 176 mg/l Aminosalicylsäure, die sich als Intermediär akkumulierte.

### Beispiel 2

Eine Lösung von 500 mg/l 6-Amino-2-naphthalinsulfonsäure in 1 000 ml Leitungswasser wird nach Zusatz von 10 ml der wässrigen Suspension der Mikroorganismenmischkultur, bestehend aus ursprünglich
40 % Pseudomonas desmolytica der Stämme DSM 5779 und DSM 6738
10 % Flavobacterium devorans (DSM 5773)
30 % Bacillus cereus (DSM 5766) und
20 % Bacillus circulans, (DSM 6983)
und Zusatz von 10 mg/l KH₂PO₄ sowie Spuren von CaCl₂ und FeCl₃ bei pH = 7 und 25 °C in einem Erlenmeyer-Kolben unter Verwendung eines Magnetrührers derart belüftet, daß das Reaktionsmedium einen Gehalt an gelöstem Sauerstoff von DO = 2 bis 4 mg/l O₂ aufweist. Nach 5 h beginnt sich der Kolbeninhalt rot zu verfärben und weist nach 10 h eine tiefrote Verfärbung auf. Das UV-Spektrum des Mediums weist ein Adsorptionsmaximum bei 240 Nm auf.

### Beispiel 3

Die gleiche Lösung wie in Beispiel 1 wird nach Zusatz von 10 ml der in Beispiel 1 beschriebenen Mischkultur derart belüftet, daß dabei nur eine gelöste O₂-Konzentration von DO = 0,4 bis 0,6 mg/l aufrechterhalten wird. Nach 10stündiger Behandlung wird die Reaktion gestoppt und das Reaktionsprodukt analysiert. Dieses Produkt ist im Gegensatz zu Beispiel 1 farblos. Das UV-Spektrum weist ein Adsorptionsmaximum bei 205 Nm auf. Die Lösung enthielt 102 mg/l NO₃⁻.

### Beispiel 4

10 ml der gleichen Mischkultur wie in Beispiel 1 werden mit 1 000 ml einer Lösung von 500 mg/l 1,3,6-Naphthalintrisulfonsäure versetzt. Nach 240 h Belüftung bei einem Gehalt an gelöstem Sauerstoff von DO = 2 bis 4 mg/l O₂ hat sich das Medium nicht werfärbt. Eine HPLC-Chromatographie ergab im Medium noch 435 mg/l 1,3,6-Naphthalintrisulfonsäure.

### Beispiel 5

Die gleiche Lösung und Mischkultur wie in Beispiel 3 mit 1,3,6-Naphthalintrisulfonsäure als Substrat wurde mit 100 mg/l (NH₄)₂HPO₄ versetzt und mit DO = 1 mg/l belüftet. Nach 240 h ergab die HPLC-Analyse im Medium 120 mg/l der Ausgangssubstanz. Das Medium enthielt außerdem 66 mg/l NO₃⁻. Dies bedeutet, daß das Substrat zum größten Teil abgebaut (mineralisiert) ist.

## Patentansprüche

1. Mikroorganismenmischkultur in fester oder flüssiger Form bestehend aus den Stämmen:
1.1 Pseudomonas desmolytica
1.2 Bacillus cereus
1.3 Bacillus circulans und
1.3 Flavobacterium devorans

2. Mikroorganismenmischkultur nach Anspruch 1, dadurch gekennzeichnet, daß es sich bei Pseudomonas desmolytica um die bei der Deutschen Sammlung von Mikroorganismen und Zellkulturen (DSM) in 38124 Braunschweig hinterlegten Stämme:
2.1 Pseudomonas desmolytica, gemäß Hinterlegung unter der Nummer DSM 5779 und
2.2 Pseudomonas desmolytica, gemäß Hinterlegung unter der Nummer DSM 6738 handelt.

3. Mikroorganismenmischkultur nach Anspruch 1, dadurch gekennzeichnet, daß es sich bei Bacillus cereus um den bei der DSM hinterlegten Stamm Bacillus cereus, Nummer DSM 5766, handelt.

4. Mikroorganismenmischkultur nach Anspruch 1, dadurch gekennzeichnet, daß es sich bei Bacillus circulans um den bei der DSM hinterlegten Stamm Bacillus circulans, Nummer DSM 6983, handelt.

5. Mikroorganismenmischkultur nach Anspruch 1, dadurch gekennzeichnet, daß es sich bei Flavobacterium devorans um den bei der DSM hinterlegten Stamm Flavobacterium devorans, Nummer DSM 5773, handelt.

6. Mikroorganismenmischkultur nach Anspruch 1 bis 5, dadurch gekennzeichnet, daß der prozentuelle Anteil der beiden Pseudomonas desmolytica in der Mischkultur 35 % bis 60 %, bevorzugt 40 % bis 55 %, besonders bevorzugt 45 % bis 50 %, der Anteil an Bacillus cereus und Bacillus circulans in der Mischkultur 25 % bis 50 %, bevorzugt 30 % bis 45 %, besonders bevorzugt 35 % bis 40 % und
der Anteil an Flavobacterium devorans in der Mischkultur 5 % bis 20 %, bevorzugt 6 % bis 15 % sowie besonders bevorzugt 7 % bis 12 % beträgt.

7. Verfahren zum mikrobiellen Abbau von substituierten Aromaten und deren Abkömmlingen, insbesondere von substituierten Arylmono-, Aryldi- und Aryltrisulfonsäuren bzw. deren Kondensationsprodukten, dadurch gekennzeichnet, daß das Substrat in Gegenwart der erfindungsgemäßen Mischkultur gemäß Anspruch 1 bis 6, bei Temperaturen von 5 °C bis 35 °C bei pH-Werten von 6,6 bis 8,2 und Sauerstoffkonzentrationen von 0,2 bis 2,5 mg/l behandelt wird.

8. Verfahren nach Anspruch 1 bis 7, dadurch gekennzeichnet, daß die erfindungsgemäße Mischkultur in einem separaten Bioreaktor gezüchtet und von dort aus kontinuierlich oder halbkontinuierlich in das Belebungsbecken der Kläranlage dosiert wird.

9. Verfahren nach Anspruch 1 bis 8, dadurch gekennzeichnet, daß gleichzeitig mit dem Abbau von Aryl-Verbindungen eine Nitrifikation von Amino-, Ammoniumstickstoff und bis zum Nitrat durchgeführt wird.
